# EUROPEAN PATENT APPLICATION

(11) **EP 1 964 933 A1**
(43) Date of publication of application: **03.09.2008**
(21) Application number: 08151783.1
(22) Date of filing: 22.02.2008
(51) Int. Cl.: C12Q 1/68, G01N 27/30, G01N 33/543

(54) **Electrochemical sensor, kit comprising said sensor and process for the production thereof**

(30) Priority: 28.02.2007 IT MI20070393
(71) Applicant: Nano-Sims S.r.l., 20123 Milano (MI) (IT)
(72) Inventor: Estini, Fabrizio, 35100, Padova PD (IT)
(74) Representative: Montelatici, Linda Anna

(57) **Abstract**

The present invention relates to an electrochemical sensor for the detection of biological molecules and a process for the production thereof. In the sensor, an electrode system comprising at least one working electrode (1), one counter electrode (3) and a reference electrode (4) operate inside of a reaction camber (18). The reaction chamber (18) is intended to contain a system of reagents in solution, comprising at least one electrochemical mediator. Said working electrode is made of series of nanoelectrodes (11,12) of a noble metal, inserted in an isolating layer (10) consisting of a resin suitable for lithography and nano-lithography, functionalised or functionalisable for the binding to biological molecules to be detected. The invention further refers to a kit comprising said sensor and a process for the production thereof.

## Description

The present invention refers to a nanostructured electrochemical sensor for the detection of biological molecules of various types, a kit containing said sensor and a process for production thereof.

It is known that the quantitative detection of biological molecules can be obtained by means of the technology of micromatrices or microarrays, used to investigate the expression profile of a gene or to identify the presence of a gene or of a short sequence in a mixture. A micromatrix is a miniaturised analysis device comprising a solid support on which a plurality of probes are arranged, which are formed of DNA fragments having a known sequence. To determine if a biological sample contains a certain nucleic acid, a suitable amount of RNA is extracted from the sample, that, by the use of an enzyme called reverse transcriptase, is converted in complementary DNA, which at the same time is labelled with a fluorescent molecule.

Subsequently, the DNA labelled in this way is brought into contact with the probes of the micromatrix to allow the hybridisation. The DNA binds to a specific probe and can be identified by simply detecting the position where it has been bound. The image of the micromatrix is acquired and processed by a computer in order to evaluate the colour and the luminosity of each spot, which vary as a function of the type and the quantity of the complementary DNA binding to a specific probe.

Therefore, the quantitative measurement of DNA obtained by means of micromatrices is only approximate. Further, the preparation of these devices for the analysis is extremely laborious because it is also necessary to produce the marking of the DNA sample with fluorescent molecules.

An electrochemical method for the quantitative measurement of DNA is described in the publication "Electrochemical Quantitation of DNA Immobilized on Gold" Anal. Chem. 1998, 70, 4670-4677. In this method, DNA probes of known sequence are bound to a gold electrode through bidentate molecules, for example of 6-merdcapto-1-hexanol. The probes are then put into contact with a hybridisation solution containing DNA of unknown sequence and subsequently with an electrolyte of low ionic strength containing a redox cationic marker. Such a marker exchanges with the counterions associated to the nucleotidic residue of the DNA. Then the quantity of the marker, which is proportional to the number of nucleotidic residues and can be correlated to the surface density of the hybridized DNA, is measured by coulometry. In this way it is possible to qualitatively and quantitatively measure the DNA present in the hybridization solution.

However, a disadvantage of said electrochemical method is that the binding of DNA molecules to the electrodes leads to a change of the working conditions of the electrodes and results in a reduction of the measured current, with a disadvantage of the efficiency of the electrode.

Also known are electrochemical biosensors in which the measurement of a specific analyte in a sample is performed due to the redox reaction that takes place between said analyte and an electrochemical mediator, in the presence of a redox enzyme specific for said analyte. The electrochemical mediator is then set back to the original oxidation state by contacting the electrode, and the concentration of the analyte in the sample is determined in function of the measured current value.

For example, in EP1118675 a biosensor of this type is described, which includes a measurement electrode and a counter electrode, realized by moulding, on a base plate protected by a cover element. Between said base plate and said cover element there is a duct for the introduction of the sample solution into the biosensor. Said duct houses in turn a solid support for a system of reagents including said electronic mediator and said redox enzyme, that are released from the support when the sample solution is introduced into the biosensor and dissolves in said solution.

Among the analysis that can be performed by the known biosensor, those are mentioned for which suitable enzymes are available, such as the measurement of glucose in blood or of cholesterol in serum.

However, said document does not mention the possibility to perform qualitative and quantitative analysis of nucleic acids, nor does is disclose a nanostructured sensor for this type of analysis.

Therefore, object of the present invention is to provide an electrochemical nanostructured sensor that is free of said disadvantages and a process of the production thereof. Said object is achieved by an electrochemical sensor, whose main features are specified in claim 1, a kit comprising said sensor, whose features are specified in claim 17, and a production process whose features are specified in claim 17. Other features of the sensor, the kit and the process are specified in the dependent claims.

An advantage of the sensor according to the present invention is that it allows to perform qualitative and quantitative analysis of any electroactive biological molecule, for example nucleic acids, proteins or enzymes. Therefore, the sensor according to the present invention finds its applications as well in the field of fundamental research as well as in medical diagnostics, particularly for genetic diseases, where the genetic expression of healthy cells is compared to that of cells affected by the disease under investigation.

Another advantage of the sensor according to the present invention is that the use thereof is very simple and does not require the marking of the analyte by fluorescent or radioactive molecules.

Even a further advantage of the sensor according to the present invention consists in its analysis accuracy and precision and its high signal to noise ratio, which are due to the nanometric dimensions of the electrodes and to the presence of a plurality of nanoelectrodes connected among each other.

A further advantage of the sensor according to a preferred embodiment of the invention is that the detection of a specific biological molecule is indicated also by means of an optical signal that adds up to the electrochemical signal, increasing in this way the efficiency of the sensor.

Further advantages and characteristics of the sensor according to the present invention will appear to those which are skilled in the art from the following detailed description of an embodiment thereof with reference to the attached figures, wherein:
- Fig. 1 is a simplified representation of the complex of electrodes of the sensor according to a preferred embodiment of the present invention;
- Fig. 2 shows an enlarged schematic view of the configuration of the working electrodes of the sensor according to a preferred embodiment of the present invention;
- Fig. 3 shows a schematic representation of the structure of the working electrodes of Fig. 2;
- Fig. 4 is a operation scheme of a working electrode of the sensor according to the present invention;
- Fig. 5 shows a cross-sectional lateral view of the central part of the sensor of Fig. 2;
- Fig. 6 shows a top view of a covering element of the sensor according to Fig. 2;
- Fig. 7 shows a cross-sectional lateral view of the central part of the sensor according to a second embodiment of the invention;
- Fig. 8 shows an overall cross-sectional view of the sensor of Fig. 2; and
- Fig. 9 shows a top view of the sensor of Fig. 8.

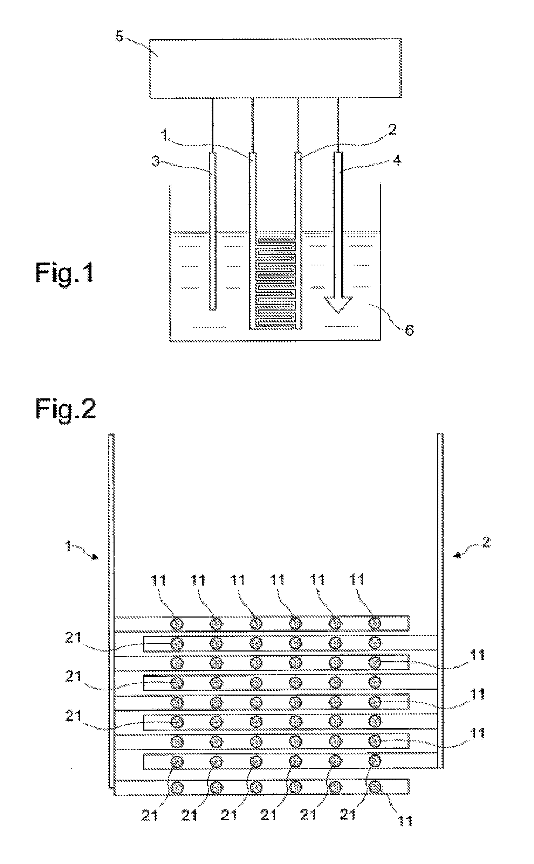

The sensor according to the invention includes in a known way a system of electrodes comprising at least a working electrode, a counter electrode and a reference electrode connected to a potentiostate. With reference to figure 1 there is shown that, according to a preferred embodiment, the sensor according to the invention includes two working electrodes 1 and 2; a counter electrode 3 and a reference electrode 4, connected to a bipotentiostate 5. Said bipotentiostate 5 controls independently the four electrodes, operating on the two working electrodes according to a criteria that will be illustrated in the following.

When the sensor is in use, said electrodes 1, 2, 3 and 4 are immersed into a solution 6 which can be aqueous or also composed of ionic liquids or aprotic solvents having a low dielectric constant, for example acetonitrile. Such solvents may be necessary for special types of analysis because they allow to extend the accessible potential window and to perform an analysis of species with high oxidation potential or of those having a negative reduction potential.

With reference to figure 2 it can be seen that the working electrodes 1 and 2 are composite electrodes, i.e. composted of series of nanoelectrodes arranged on an isolating substrate.

In particular, in the embodiment shown here, the electrodes 1 and 2 form an array having a linear interdigitated arrangement, each of them including various linear series of nanoelectrodes, indicated with the reference numbers 11 for electrode 1 and 21 for electrode 2. In the present description and in the claims, the portion of the working electrodes that includes the series of nanoelectrodes is defined as active area of the electrodes.

In other embodiments of the invention it can be favourable to use other interdigitated arrangements of the electrodes, for example, circular, triangular or asymmetric. Also the shape of the nanoelectrodes could be different from the circular shape illustrated in the figure, for example, they could have an oval shape and variable dimensions.

Each of the series of interdigitated electrodes does not necessarily contain only one row of nanoelectrodes, but instead one or more aligned or staggered rows of nanoelectrodes can be provided, which define the so-called "array of array" configuration. Further the nanoelectrodes must not necessarily be arranged in a regular way, but they can also form disordered or partially disordered arrays, so-called "ensemble".

In fact it is known in electrochemistry that the use of composite electrodes, these being composed by interdigitated series of nanoelectrodes having smaller dimensions than the thickness of the diffusion layer, results in an increase of the diffusion flux and therefore in an amplification of the signal with respect to a signal measured at a single macroscopic electrode. Consequently an improvement of the signal to noise ratios of the sensor is obtained.

Particularly for the electrodes with an interdigitated arrangement an increase of the signal is obtained also thanks to a catalytic effect on the oxidation and reduction reactions of the analyte, due to the mutual proximity of the interdigitated electrodes.

Further, the nano-structured electrodes have the advantage to enable an increase of the mass transport of the species involved in the electrochemical reactions towards the electrodes and a consequent acceleration of the electrochemical reactions themselves. In fact, for macroscopic electrodes the mass transport is controlled only by the diffusion of the reactive species at the electrodes. Consequently the velocity of the electrochemical reaction is limited. Instead, on nano-structured electrodes the mass transport is highly accelerated due to the nanometric arrangement that allows an additional diffusive component in radial direction of the single nanoelectrodes, which is not present in macroscopic electrodes.

A further advantage of the nano-structured electrodes is the variation of the electrochemically active surface with respect to that of macroscopic electrodes. Such variation determines a very small time constant for the RC circuit of the analytic system and therefore allows high scanning velocities. For a complete description of the properties and the advantages of composite electrodes with interdigitated structure it is referred to the wide literature of the field.

In order to allow for the above-mentioned effects, the electrodes must operate in condition of total overlap of the diffusion layers of each single nanoelectrode, therefore the density of the nanoelectrodes on the various bands must be high. Also the distance between neighbouring series of nanoelectrodes must be very small, preferably this must be smaller than about 10 µm. The width of the part of the isolating substrate carrying each row is preferably smaller than about 1.2 µm, while the size of the nanoelectrodes formed on this portion is nanometric. Preferably their diameter is smaller than about 0.6 µm.

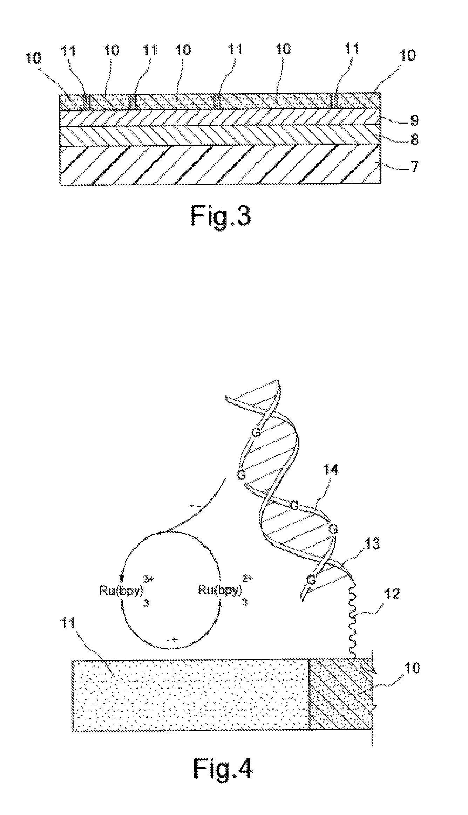

In figure 3 it can be seen that the working electrode 1, analogously to electrode 2, includes an isolating substrate 7 on which a first metallic layer 8, formed of a conducting metal or alloy, is deposited. This first layer has the function to modify the surface of the isolating substrate 7, enabling the adhesion of a second metallic layer 9 of a noble metal suitable to realize the nanoelectrodes, for example gold.

Isolating materials suitable for the realization of the substrate for the working electrodes of the sensor according to the present invention are, for example, glass, alumina or polymers having a high dielectric constant, such as a polycarbonate.

The material of the first metallic layer 8 must be chosen in function of the special nature of the layer. Preferably, said isolating substrate is made of glass, onto which a first metallic layer of titanium is deposited.

Onto said second metallic layer 9 the nanoelectrodes 11 are made, formed preferably of the same noble metal as the second layer, these electrodes result inserted into an isolating layer 10 formed of a functionalised or functionalisable resin, suitable for lithography or nano-lithography. In the present description and in the claims, functionalisable resin means a resin provided with functional reactive groups, that is suitable to form, under special reaction conditions, a stable bond to a plurality of molecules 12 in function of connection between said isolating layer 10 and a probe 13 specific for a determined biological molecule 14 to be detected. Instead, functionalised resin means a resin on which surface molecules 12 are already bound, having the above defined function.

In general, said functionalisable resin can be formed by any polymer or copolymer provided with free reactive groups, such as for example amino, carbonylic, carboxylic groups and substituted or heterocyclic aromatic rings. Said resin is preferably selected from the group formed of: polycarbonates, polymethylmetacrylate, polyvinylpyridine, polystyrene and their derivatives or copolymers. For example, copolymers of polymethylmetacrylate with polyvinyl acetic acid, polyvinylpyrrolidone, polyvinyl alcohol or polystyrene can be used, or copolymers of polystyrene with polyvinyl acetic acid, polyvinyl pyridine, or polystyrene with amino, carboxylic or phenolic groups substituted in the aromatic ring. Even more advantageously a photosensitive resin as the resin Novolac can be used.

In figure 4 it is shown that the surface of the isolating layer 10 is already suitably functionalised for the binding to biological molecules to be detected. In particular, on the surface of the isolating layer a plurality of molecules 12 are bound that act as connection between said isolating layer 10 and a probe 13 specific for a determined biological molecule 14 to be detected.

Therefore said molecules 12 are bidentate, that are bound to said isolating layer 10 thanks to the reaction with said reactive functional groups of said layer, and are further provided with other reactive functional groups complementary to the probe. These molecules 12 further contain a sufficiently long linear chain to allow the probe to arrange itself at a certain distance from the surface of the resin, allowing in this way the attachment of a higher number of analyte molecules in the neighbourhood of each nanoelectrode, thus overcoming the hindrance created by the considerable size of certain analytes. Preferably, said linear chain contains at least six atoms.

For example, for the connection of a probe consisting of a set of nucleic acids of known sequence, the molecules 12 can advantageously contain aminic, epoxidic, carboxylic, pyrrolidonic, halogenic functional groups, reactive with the DNA strand of the probe, suitably functionalised with groups suitable to react with said functional groups of the molecules 12.

Preferably, said molecules 12 are selected from the group of silanes. Even more preferably, for the functionalisation of the surface of the isolating layer 10 3-(triethoxysilil)-propylamine or 3-(glycidoxypropyl)-trimethoxisilane is used. In fact these molecules have a very good stability and their final functional groups can be substituted, also in a step following the production step of the sensor, by any other terminal group suitable for the attachment of a specific probe.

Further, the molecules 12 lack functional groups capable of forming a binding with the surface of the nanoelectrodes. For example, in the case that the nanoelectrodes are realized from gold, the molecules 12 may not contain mercaptanic groups

In figure 4 it also can be seen that in the sensor the surface of the electrodes and the neighbouring surface of the isolating layer 10 are in contact with a solution containing an electrochemical mediator, specific for a certain biological molecule 14 to be detected. Said mediator acts by transferring electrons between said biological molecule 14 bound to the probe 13 and a neighbouring nanoelectrode 11. For this purpose it is necessary that the electrochemical mediator has a suitable redox potential which makes it suitable to exchange electrons during the analysis with molecules of nucleic acids or other electroactive biological molecules. In case it is necessary to detect various biological molecules, a mixture of different chemical mediators can be used.

According to an alternative embodiment of the invention, the molecules 12 can allow the attachment of an electrochemical mediator suitably functionalised.

Among the various types of electrochemical mediators known in the field, the use of (ferrocenylmethyl)trimethylammonium hexafluorophosphate or of tris(2,2'-bipyridyl)ruthenium(II) chloride hexahydrate, osmium bipyridyle or substituted homologs is preferable. In fact, these electrochemical mediators also have a property of electrochemical luminescence in relation to the transition between two different oxidation states, therefore by suitably preparing the sensor, as illustrated in the following, it is possible to measure its light emission, thereby obtaining a further signal that can be correlated to the presence and the quantity of a certain analyte. In alternative, other electrochemical mediators con be used, for example methylene blue or potassium ferrocyanide.

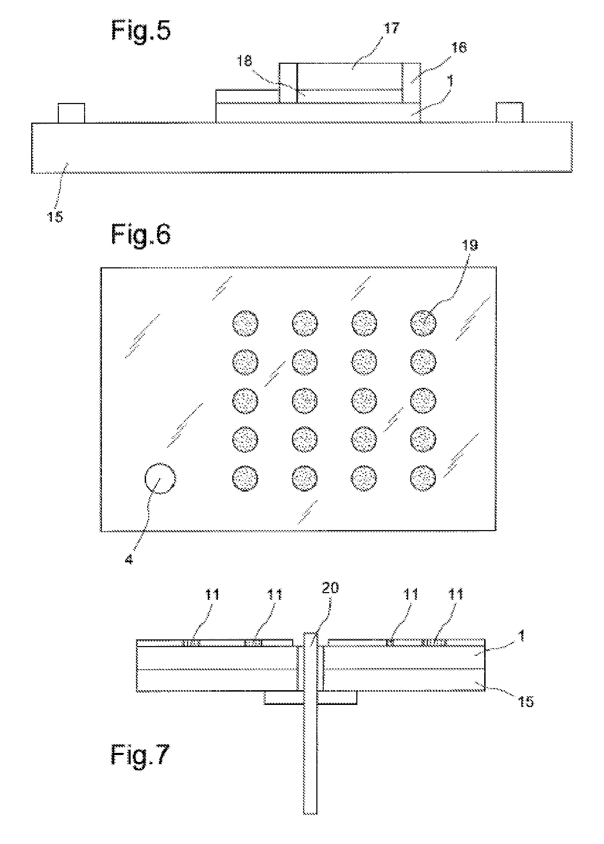

With reference to figure 5, there is shown that the working electrodes 1 and 2, so far described, are connected over a base 15 of a container for integrated circuits of the type DIL (dual in line). Above the electrodes a frame 16 of inert polymeric material is arranged, that supports a covering element 17. Thus a reaction chamber 18 is defined inside the sensor, intended to contain the solution to be analysed which is injected into the chamber and filled through two small openings of the frame 16. It must be noted that the frame surrounds only the active parts of the electrodes 1 and 2, leaving the ends uncovered.

According to a preferred embodiment of the invention, shown in figure 5 and 6, the acquisition of the optical signals for recognizing an analyte, sent from the electrochemical mediators, is carried out thanks to a matrix of photodiodes enclosed in said covering element 17. For this purpose, said element is formed at least partially of a material optically transparent to the light signal emitted by said mediators.

Preferably, covering element 17 includes at least one layer of optical transparent and isolating material, facing the outside of said reaction chamber, and one layer of optical transparent and conducting material, facing the inside of chamber 18. For example, the optical transparent and isolating material of the external layer can be a polycarbonate, while the optically transparent and conducting material of the layer on the inside of chamber 18 is preferably from mixed oxide of tin and indium (ITO). The photodiode matrix is enclosed between the two layers.

In figure 6 it can be seen, protected inside of the covering element, a photodiode matrix 19, whose positions coincides with that of the nanoelectrodes arranged inside of chamber 18. Each photoelectrode is suited to measure the optical signals sent from the electrochemical mediator in correspondence of a nanoelectrode.

The acquisition of the optical signal can also be made by means of other types of optical sensors, as for example CCD sensors.

According to an alternative embodiment of the invention, represented in figure 7, the acquisition of the optical signal is instead obtained by means of an optical fibre 20 having a suitable pass-band, which can be introduced into chamber 18 through a suitable opening in the isolating substrate 7 and the base 15, and is fixed to these elements. The optical fiber 20 collects the light signal emitted by the electrochemical sensor and feeds the signal, also in this case, to a suitable optical sensor, for example, a photodiode or a CCD sensor.

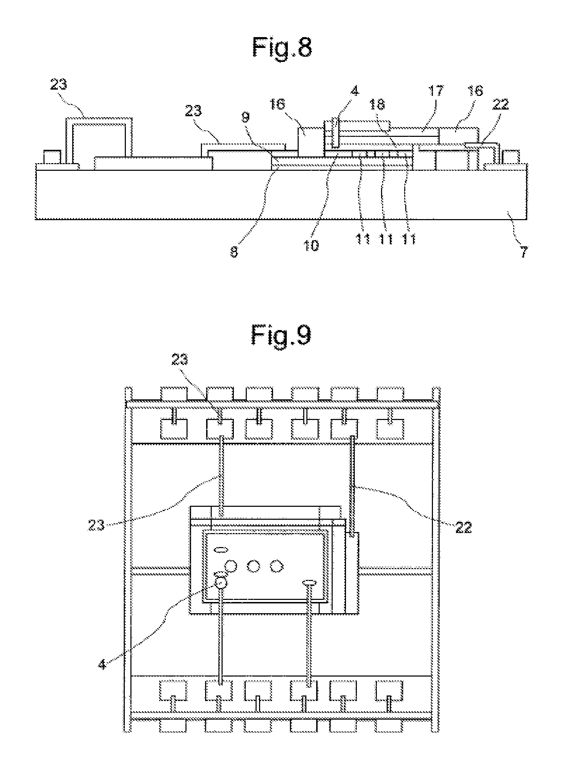

Figures 8 and 9 show also a reference electrode 4, which according to the present embodiment of the invention is an Ag/AgCl electrode supported by the covering element 17. In fact, in said covering element a channel is formed that connects chamber 18 to the outside and that houses said reference electrode. In this way the reference electrode is inserted with one end into chamber 18, while the other end is connected to bi-potentiostat 5 (not shown).

With reference to figure 8, there is shown that in the sensor according to a preferred embodiment of the invention, a conducting side of the ITO layer of the covering element 17 acts as counter electrode and also this is connected, by means of connection 22, to the bi-potentiostat. The other side of the ITO layer is not a conductor and thus acts as transparent cover of the photodiode matrix 19.

Other connections 23, attached to the terminals of the working electrode, are connected to said bi-potentiostat.

The electrochemical measurements can be performed by means of the sensor according to the present invention, by operating in the following way.

Suitably functionalised single-strand DNA probes, or other probes specific for the biological molecules to be detected, which bind to the free ends of the connecting molecules 12, are deposited at determined sites of the surface of isolating layer 10 of the working electrodes 1 and 2, close to the nanoelectrodes,. The modality and the conditions for the deposition depend on the nature of said connecting molecules 12 and are easily determinable for those skilled in the art.

According to an alternative embodiment of the sensor according to the present invention, as illustrated in the following, the sensor is already provided with DNA probes bound to the connecting molecules 12 in certain sites of the surface of the isolating layer 10 of the working electrodes 1 and 2, therefore this first step is not necessary.

Subsequently, a DNA extract to be analysed is prepared. For example, with the sensor according to the present invention an extract of human cells, of virus or bacteria obtained according to known methods can be analyzed.

With a hybridisation procedure, the DNA molecules of the extract are bound to those complementary to the probe to form double-chain DNA, which result immobilized at the sites of the layer 10, close to the nanoelectrodes.

Then into the sensor a buffered solution is injected, containing an electrochemical mediator suitably chosen in function of the special analysis to be performed.

At this point bi-potentiostat 5 is activated that imposes a fixed potential to one of the working electrodes and a variable potential to the other one. For example, on the working electrode 1 or generator a linear scan of the potential can be operated. The applied potentials depend on the nature of the analysed molecules, on the specific electrochemical mediator used and on the specific type of the electrode. For example, the potential of electrode 1 or generator can linearly vary from 0 to 0.6 V, while electrode 2 or collector can be held at a potential that is 0.1 V higher than the redox potential of the analysed species.

In case that the sensor includes only one working electrode, a classical cyclic voltammetry operating in single modality can be performed, according to principles know in art.

Based on the registered current the qualitative and quantitative measurement of the wanted species is performed. Contemporarily, it is possible to measure with the photomultiplier the light emitted from the electrochemical mediator. In this way, a second signal is obtained, also this being related to the quantity of the various analysed biological molecules. Obtaining two types of signals, electrochemical and optical, allows to further increase the efficiency and the precision of the sensor.

The sensor according to the present invention can be built according to a process including the deposition of a first metallic layer 8 of a conducting metal onto an isolating substrate 7, and a subsequent deposition of a second metallic layer 9 of a noble metal on said first metallic layer 8.

The deposition of the metallic layers can be performed, for example, by means of metal evaporation under vacuum. Alternatively, it is possible to use techniques of electrodeposition or reduction deposition (also known as "electroless"), known in the field.

Then, on said second metallic layer 9 an isolating layer 10 is deposited, preferably of photosensitive resin. To perform this, it may be preferable to deposit preventively a layer of tackifying material by using the spin coating technique.

Onto said layer the nanoelectrodes 11, 21 are formed, which can have a uniform or non-uniform distribution, according to the necessities of the analysis. In particular, the isolating layer 10 undergoes a suitable lithographic or nano-lithographic technique, as for example optical high resolution lithography, laser, X-ray, electronic, immersion or imprinting lithography, with the help of a common graphic programme for lithographic processes, thanks to which it is possible to define the shape and the distribution of the nanoelectrodes to be realized. A selective developing step of the resin that forms the isolating layer is necessary for some of these techniques.

In this way a plurality of recesses are obtained in the polymeric layer 10, having such a depth to expose the underlying second metallic layer. Inside said recesses, the nanoelectrodes are formed of the same noble metal as the second metallic layer by electrolytic growth in a bath at constant potential or by electroless deposition.

It is advisable to clean the surface of the just formed nanoelectrodes, for example, by means of immersion of the device in an acid solution. The cleaning of the nanoelectrodes can then be completed, after having finished the electrical connection thereof, by applying some hundred low voltage potential scans to the electrodes.

Before carrying out the functionalisation of the surface of the polymeric resin, the electrodes should preferably undergo testing by subjecting them to some scanning cycles of the potential in the presence of a solution of a reference electrochemical mediator, for example tris(2,2'-bipyridyl)ruthenium(II) chloride hexahydrate.

Said functionalisation of the isolating layer 10 is preferably carried out using the technique of chemical vapour deposition (CVD) of the desired reagent, which varies according to the biological molecule to be analysed. The technique of chemical vapour deposition is advantageous as it is simple, economic, allows an easy control of the surface density of molecules 12 an does not include undesired phenomena of reagent decomposition or the formation of by-products. However, other known techniques could be used for the functionalisation of the isolating layer, allowing the binding of suitable connecting molecules 12. Optionally, the functionalisation can be carried out by the final user of the sensor.

At this point, the substrate can be prepared for the acquisition by means of optical fibers of the light signal possibly emitted at the electrodes. Thus, an optical fibre of suitable pass-band is fixed to the beforehand-perforated substrate, as shown in figure 7. In case that the acquisition of the optical signal is carried out by means of photodiodes arranged in the covering element 17, the use of optical fibers is not necessary.

The working electrodes realized and fuctionalised in this way are then fixed on a base 15 of a container for integrated circuits, for example of the DIL type. The electrodes are then connected in a known way to the electrical contacts prepared on the container.

Subsequently, the reaction chamber 18 is created, by arranging a frame 16, made of a polymeric, inert material and having two small openings, around the active portions of the working electrodes. On the contrary, the ends of working electrodes 1 and 2 are left outside of frame 16.

Covering element 17, in which a reference electrode is integrated, for example Ag/AgCl, is laid on the frame. According to an embodiment of the invention, this covering element 17 includes at least one layer of an optically transparent and isolating material, for example polycarbonate, one layer of conducting material of mixed oxide of tin and indium (ITO) that acts also as counter electrode, and a matrix of photodiodes that is enclosed between said two layers.

Also the ends of the reference electrode and of the counter electrode are connected to the contacts provided in the container.

The following example illustrates the using modalities of the sensor according to the invention.

### EXAMPLE

Two electrodes according to the invention of 200 x 200 µm were cleaned with water, incubated in a just prepared solution of 2.5% (v/v) of glutaraldehyde in 0.1M phosphate buffer at pH 7 for 2 hours at room temperature. The electrodes were then cleaned with phosphate buffer (2 x 10 min). A solution was prepared, adding 100 pmol/mol NH₂ -ssDNA to 1 ml of phosphate buffer at pH 7, and the electrodes were incubated for 4 hours at 37°C in that solution.

Subsequently the electrodes were washed 5 times with a SSC solution containing Tween 20 at 0.1% for 15 min, and then again with deionised water.

Then, a hybridization solution was prepared, containing 20 µM complementary ss-DMA in 25 mM sodium phosphate buffer solution at pH 7, 25 mM in NaCl and 100 mM in MgCl₂. The electrodes were incubated for 40 min at 37°C. Then, two rinsings with phosphate buffer at pH 7, for 10 minutes, and three rinsings with water were carried out.

Then, a solution of 30uM Ru(BPy)₃ in phosphate buffer of pH 7, containing also sodium oxalate at a 1000 times lower concentration with respect to Ru(BPy)₃ was introduced into the reaction chamber of the sensor.

A voltagram was performed by varying the potential in the interval of -0.5 V and + 0.8 V at a scanning velocity of 20mV/s with a counter electrode and a SCE reference electrode.

Simultaneously, the light emitted from Ru(BPy)₃ was measured by the photomultiplyer.

In this way an electric signal, coming from the photomultiplyer by virtue of the electrochemical luminescence generated by the used electrochemical mediator, and a second electric signal generated from the voltametric scan at a potential, specific for the used electrochemical mediator, were obtained. The variation of the difference of the measured current intensity, with respect to the current generated from the presence of the electrochemical mediator alone provided, by previous calibration, data related to the quantity of electroactive molecules analysed by the sensor.

## Claims

1. Electrochemical sensor for the detection of biological molecules, comprising a system of electrodes operating inside of a reaction chamber (18) which is intended to contain a system of reagents in solution, said system of electrodes comprising at least one working electrode (1), one counter electrode (3) and one reference electrode (4), said system of reagents comprising at least one electrochemical mediator, **characterised in that** said working electrode (1) is formed of series of nanoelectrodes (11, 21) of a noble metal inserted into an isolating layer (10) made of a resin suitable for lithography and nano-lithography, said resin being functionalised or functionalisable for the binding to biological molecules to be detected.

2. Sensor according to claim 1, **characterized in that** said system of electrodes includes two mutually interdigitated working electrodes (1, 2).

3. Sensor according to claims 1 and 2, **characterized in that** a plurality of molecules (12), acting as a connection to a probe (13) specific for a biologic molecule (14) to be detected, are bound to the surface of the isolating layer (10).

4. Sensor according to claim 1, **characterized in that** said resin is a photosensitive resin.

5. Sensor according to claim 1, **characterized in that** said resin is selected from the group formed of: polycarbonate, polymethylmethacrylate, polyvinyl pyridine, polystyrene, derivatives and copolymers thereof, and phenol-aldehydic copolymers.

6. Sensor according to claim 1, **characterized in that** said connecting molecules (12) are 3-(triethoxysilil)-propylamine or 3-(glycidoxypropyl)-trimethoxysilane.

7. Sensor according to claim 2, **characterized in that** said working electrodes (1, 2) form an array having a symmetrical linear interdigitated geometry.

8. Sensor according to claim 2, **characterized in that** each of said working electrodes comprises an isolating substrate (7), a first conducting metallic layer (8) and a second metallic layer (9), made of the same noble metal as the nanoelectrodes (11, 21), on which said isolating layer (10) is deposited.

9. Sensor according to claim 1 or 2, **characterized in that** said reaction chamber (18) is provided with a covering element (17) which encloses a matrix of photodiodes (19) and is at least partially made of a material being optically transparent to the light signal emitted by said electrochemical mediator.

10. Sensor according to the previous claim, **characterized in that** said covering element (17) includes at least one layer of an optically transparent and isolating material and one layer optically transparent and conducting material facing the inside of said reaction chamber (18) and forming said counter electrode (3).

11. Sensor according to the previous claim, **characterized in that** said optically transparent and conducting material is a mixed oxide of tin and indium (ITO).

12. Sensor according to the claim 1 to 8, **characterized in that** said optical signal is acquired inside of the chamber (18) by means of an optical fibre (20) of a suitable pass-band, which feeds the signal to a suitable optical sensor.

13. Sensor according to one of the previous claims, **characterized in that** it is implemented in a DIL container.

14. Sensor according to one of the previous claims, **characterized in that** suitably single-strand DNA functionalised probes are bound to the connecting molecules (12).

15. Kit comprising a sensor according to the claims 1 to 14 and a vial containing an aqueous solution of an electrochemical mediator specific for a certain biological molecule to be detected.

16. Kit according to the previous claim, **characterized in that** said electrochemical mediator is selected from the group formed of (ferrocenylmethyl)trimethylammonium hexafluorophosphate, tris(2,2'-bipyridyl)ruthenium(II) chloride hexahydrate, osmium bipyridyle and derivatives thereof.

17. Process for the production of a sensor according to the claims 1 to 14, comprising the steps of:
a) deposition of a first metallic layer (8) of a conducting metal on an isolating substrate (7);
b) deposition of a second metallic layer (9) of a noble metal, on said first, metallic layer (8);
c) deposition on said second metallic layer (9), of an isolating layer (10) formed of a resin suitable for lithography and nano-lithography, functionalised or functionalisable for the attachment of biological molecules to be detected; and
d) formation of nanoelectrodes (11, 12) on said isolating layer (10).

18. Process according to the previous claim, **characterized by** comprising a further step of:
e) functionalisation of the isolating layer (10).

19. Process according to the previous claim, **characterised in that** said isolating layer (10) is formed from a photosensitive resin and that the formation of nanoelectrodes (11, 21) is carried out by exposure and selective development of said photosensitive resin, with formation of recesses of such a depth as to uncover the underlying second metallic layer (9) and by subsequent electrolytic growth of a noble metal inside of said recesses.

20. Process according to the previous claim, **characterised in that** the deposition of said first and second metallic layer is carried out by evaporation under vacuum or by electro-deposition of the metal.

21. Process according to claim 18, **characterised in that** said functionalisation of the isolating layer (10) is obtained by means of chemical vapour deposition (CVD) of the connecting molecules (12).

22. Process according to the previous claim, **characterised in that** suitably functionalised single-strand DNA probes (13) are deposited on certain sites of said functionalised isolating layer (10).

23. Use of the sensor according the claims 1 to 14 for the detection of electroactive molecules.
